# EUROPEAN PATENT APPLICATION

(11) **EP 2 868 321 A1**
(43) Date of publication of application: **06.05.2015**
(21) Application number: 13191320.4
(22) Date of filing: 03.11.2013
(51) Int. Cl.: A61K 31/56, A61P 5/46

(54) **A composition or group of compositions for inhibiting autocrine HCG production in adult human cells**

(71) Applicant: Flamina Holding AG, 6304 Zug (CH)
(72) Inventor: May, Michael, CH-6304 Zug (CH)
(74) Representative: Rutz & Partner

(57) **Abstract**

The composition for inhibitting autocrine HCG (Human Chorionic Gonadotropin) production in adult human cells, by administration of a composition containing at least one active component, said active agent being a competitively binding progesterone antagonist strongly binding to steroid receptors of human cells.

## Description

The present invention relates to a composition or a group of compositions that allow inhibiting autocrine production of HCG (Human Chorionic Gonadotropin) in post-natal cells of adult human organisms.

### BACKGROUND OF THE INVENTION

The hormone production stimulated by the producing cell itself and known as autocrine hormone production, is the key endocrine process of human pregnancy during the first eight weeks after conception.

This process of autocrine hormone secretion by embryonic cells is normally stopped before parturition. From then on, all hormone production is controlled by the brain, as is well known. Consequently, HCG is no longer required in adult human cells.

By determination, the descendants of the totipotent zygote successively lose their totipotenz and follow their predetermined determination.

Remarkably, each healthy cell has a cell-memory, retaining its own cell proliferation, a necessity for organized tissues, organs and stably differentiated cell types.

Cells need to be able to switch genes ON and OFF individually, also to coordinate cohabitation with neighbour cells.

In extremely seldom cases, the gene responsible for the HCG production, normally switched OFF, is accidentally switched ON.

It is known that the presence of HCG molecules can be an indicator of intragenic and intracellular damages. Possibly HCG molecules are able to facilitate exogenous agents to cause increased exogenous DNA damages, which over years could possibly increase and then be at the origin of a wide variety of diseases.

### SUMMARY OF TH INVENTION

Based on the foregoing, the main object of the present invention is to provide a composition or a group of compositions that allow inhibiting autocrine HCG-production in post-natal cells of human organisms.

It is another object of the invention to provide a composition or a group of compositions that allow inhibiting autocrine HCG-production in post-natal cells of human organisms at comparatively low cost and free of side-effects.

The above and other objects of the present invention are achieved with a composition according to claim 1 and a group of compositions according to claim 9.

### DESCRIPTION OF THE INVENTION

The real possibility that a gene of an adult cell reverts partially into an early embryonic state has not yet been seriously considered. This explains why no attempts to counteract such a fact, as early as possible, have been made public.

Under real life conditions, an eventual and very rare accidental start of autocrine HCG-production in an adult human cell needs several years to eventually develop a detectable disease.

Possibly an autocrine HCG producing cell can under some conditions, pervert neighbor cells, thereby spreading and accelerating the propagation of the abnormal functioning of its gene.

It could be shown, that surprisingly even a very low amount of a competitively binding progesterone antagonist is able to inhibit unwanted autocrine HCG production in adult cells, thus eliminating the risk of potential damage caused by this unwanted process.

Extrapolations of private in vitro experiments indicate that already 0.1 mg to 2 mg per kg body weight, preferably 0.5 mg to 1 mg per kg body weight and per year of an active agent are sufficient.

It has been an unexpected surprise to realize, that a progesterone antagonist is able to counteract and inhibit the autocrine HCG production of an adult cell.

A time spacing of approximately 6 to 24 months between applications showed good results in preliminary private testing.

For treatments of an aggravated deficiency of the epithelium, second carrier products, as creams, spray, suppository, transdermal patches can be useful to facilitate applications comprising 1 to 10, preferably 2 to 5 w%/W of the active agent.

Further, it has been found that compositions are applicable that have been developed for contraceptive purposes such as the compounds disclosed in [1]. US 6608074 B2. Hence, in a preferred embodiment the active agent is one or a combination out of the group as described in [1] and useful variations thereof.

As stated above the composition can be administered with an extremely low dosage which is below the implantation inhibition dose described in [1], which is enclosed herewith in its entirety.

The dosage selected according to the method disclosed in [1] is effective to inhibit the formation of endometrial glands, which glands are a requirement for the implantation of a fertilized egg in the uterus.

Hence, the dosage can be a fraction of the dosage applied for contraceptive purposes.

A monthly dosage for a person with a weight of 60kg may be in the range between 0.2 mg to 10 mg. e.g. of the compound of the active agent 11β-[(4-N,N-dimethylamino)-phenyl]-17α-hydroxy-17β-(3-hydroxypropyl)-13α-methyl-4,9(10)-gondien-3-one disclosed in [1] or another competitive progesterone antagonist.

For females the dosage could further be reduced by 10% to 50% compared to a male person of the same age. Still further, the dosage is preferably adapted to environmental, habitual and genealogical considerations. Hence, the dosage and consequently the costs and efforts for the application can be kept extremely low, while the benefit is substantial.

The absorption of the active agent is preferably increased if a pharmaceutically acceptable first carrier medium to improve the transport of the active molecules of the active agent into the cytosol of human cells is combined with/to the active agent.

Good results are achieved if the first carrier medium is a carrier protein, a cell penetrating peptide, a polar aprotic solvent such as Dimethyl sulfoxide (DMSO), a lipid, an alcohol, a permease, a liposome, a sulfur containing compound or a mixture thereof.

A safe and controllable application of the active agent is obtained, if a second pharmaceutically acceptable carrier medium is added to the composition, as for example a liquid carrier, if the active agent shall be injected, a gelatin for sublingual application, a suppository for anal, and a cream, spray or oil for mucosal or transdermal application.

Hence, inhibiting autocrine HCG-production can be performed with low dosages that exhibit no side effects. Further, costs are extremely low even for general preventive application in the public. Potential risks are avoided or significantly reduced, which could lead to high healing costs and suffering of the patients.

Preferably a group of at least a first and a second composition is provided that comprise different carrier media and that are applicable in different parts of the body for forwarding the active agent via different pathways to a predetermined application zone.

Preferably the first composition that is provided with a first concentration of the active agent and the second composition that is provided with a second concentration of the active agent are adapted to one another in such a way that the desired amount of active agent is delivered to the field of application and the concentration of the active agent along a specific pathway does not exceed specific values. The first composition is provided for example for transdermal application and the second composition is provided for inhalatory application. Alternatively the first composition is provided for transdermal application and the second composition is provided for oral application.

## Claims

1. Composition for inhibiting autocrine HCG (Human Chorionic Gonadotropin) production in adult human cells, by administration of a composition containing at least one active component, said active agent being a competitively binding progesterone antagonist strongly binding to steroid receptors of human cells.

2. Composition according to claim 1, whereby the active agent is a compound having properties required for contraceptive or abortive purposes, such as a compound disclosed in US 6608074 B2.

3. Composition according to claim 1 or 2, whereby the composition is provided in units containing the active agent with a dosage or concentration that is below the implantation inhibiting dose.

4. Composition according to claim 3, whereby the composition is provided in units containing the active agent with a dosage or concentration that is by a factor n below the implantation inhibiting dose, which factor n is in the range from 2 to 20 or higher.

5. Composition according to claim 1, whereby the composition is provided in units designed for the monthly, semi-annual or annual application of the active agent in annual dosages in the range between 0.1 mg to 2.0 mg preferably 0.5 mg to 1.0 mg per kg of person/body-weight.

6. Composition according to one of the claims 1 - 5, whereby a pharmaceutically acceptable first carrier medium to improve the transport of the active molecules of the active agent into the cytosol of human cells is combined with/to the active agent.

7. Composition according to claim 1, whereby the first carrier medium is a carrier protein, a cell penetrating peptide, a polar aprotic solvent such as Dimethyl sulfoxide (DMSO), a lipid, an alcohol, a permease, a liposome, a sulfur containing compound or a mixture thereof.

8. Composition according to one of the claims 1 - 7, whereby a second pharmaceutically acceptable carrier medium to enable and facilitate the application and absorption of the active agent is added to the composition such as a liquid carrier, if the active agent shall be injected, a gelatin for sublingual application, a suppository for anal, and a cream, spray or oil for mucosal or transdermal application.

9. Group of at least a first and a second composition according to one of the claims 1 to 8 that comprise different carrier media and that are applicable in different parts of the body for forwarding the active agent via different pathways to a predetermined application zone, whereby the first composition that is provided with a first concentration of the active agent and the second composition that is provided with a second concentration of the active agent are adapted to one another in such a way that the desired amount of active agent is delivered to the field of application and the concentration of the active agent along a specific pathway does not exceed specific values.

10. Group of at least a first and a second composition according claim 9, wherein the first composition is provided for transdermal application and the second composition is provided for inhalatory application.

11. Group of at least a first and a second composition according claim 9, wherein the first composition is provided for transdermal application and the second composition is provided for oral application.
